# EUROPEAN PATENT SPECIFICATION

(11) **EP 4 081 531 B1**
(45) Date of publication and mention of the grant of the patent: **04.12.2024**
(21) Application number: 20828682.3
(22) Date of filing: 21.12.2020
(51) Int. Cl.: C07K 7/08, C07K 14/47, A61K 38/10, A61K 38/16, A61P 27/02

(54) **A COMPOSITION COMPRISING S-ARRESTIN PEPTIDES**
ZUSAMMENSETZUNG MIT S-ARRESTIN-PEPTIDEN
COMPOSITION COMPRENANT DES PEPTIDES DE S-ARRESTINE

(30) Priority: 23.12.2019 GB 201919222
(43) Date of publication of application: 02.11.2022
(73) Proprietor: Worg Pharmaceuticals (Zhejiang) Co., Ltd., Huzhou, Zhejiang (CN)
(72) Inventor: SCHURGERS, Evelien, 3590 Diepenbeek (BE); HOEDEMAEKERS, Brecht, 3590 Diepenbeek (BE); JANSSON, Liselotte, 3590 Diepenbeek (BE)
(74) Representative: D Young & Co LLP
(86) International application number: PCT/IB2020/062285
(87) International publication number: WO 2021/130653

(56) References cited:
- WO-A1-2018/127830
- WO-A2-2016/198932
- US-A1- 2011 008 382
- MADISON KYGER ET AL: "Effective Arrestin-Specific Immunotherapy of Experimental Autoimmune Uveitis with RTL: A Prospect for Treatment of Human Uveitis", TRANSLATIONAL VISION SCIENCE & TECHNOLOGY, vol. 2, no. 2, 28 February 2013 (2013-02-28), pages 1 - 15, XP055302746, DOI: 10.1167/tvst.2.2.1

## Description

### FIELD OF THE INVENTION

The present invention relates to a composition which comprises peptides derived from S-Arrestin (retinal arrestin, S-antigen, S-Ag). The composition or peptides may be useful in the prevention and/or suppression of S-Ag autoimmunity, which is useful in the treatment and/or prevention of uveitis.

### BACKGROUND TO THE INVENTION

Uveitis describes a group of diseases associated with inflammation of the uvea. The uvea is a region of the eye located between the sclera and the retina, and includes the iris, ciliary body and choroid. The uvea provides most of the blood supply to the retina. The associated diseases are not restricted to those affecting the uvea directly, and adjacent structures such as the retina, optic nerve, lens, vitreous and sclera can be affected in manifestations of uveitis.

All forms of uveitis are characterised by an inflammatory cellular infiltrate, commonly visualised using a biomicroscope. In 2010, it was estimated that 285 million people were visually impaired; of these, 39 million were blind and it was approximated that 10% of the cases were due to uveitis. (Global data on visual impairments, The World Health Report, WHO (2010) http://www.who.int/blindness/GLOBALDATAFINALforweb.pdf).

Present treatments for uveitis include the use of glucocorticoid steroids and other immunosuppressive agents such as methotrexate.

There is, however, a need in the art for alternative treatments for uveitis. The present invention addresses this need.

WO 2018/127830 A1 describes S-Arrestin peptides and therapeutic uses thereof.

### SUMMARY OF ASPECTS OF THE INVENTION

The present inventors have found that a "cocktail" of three S-Ag peptides is particularly effective in suppressing or preventing S-Ag-specific T cell activation *ex vivo.*

Thus, in a first aspect the present invention provides a composition which comprises S-Ag peptides, wherein the S-Ag peptides consist of the following S-Ag peptides:
a peptide consisting of the amino acid sequence KKKAFVEQVANWLKKK (SEQ ID NO: 1); and
a peptide consisting of the amino acid sequence KKKLTFRRDLYFSRVQVYKKK (SEQ ID NO: 2); and/or
a peptide consisting of the amino acid sequence KKKVIFKKISRDKSVTIYLGKKK (SEQ ID NO: 3).

KKKAFVEQVANWLKKK (SEQ ID NO: 1) is also referred to as 9K1K herein. KKKLTFRRDLYFSRVQVYKKK (SEQ ID NO: 2) is also referred to as 17JK herein. KKKVIFKKISRDKSVTIYLGKKK (SEQ ID NO: 3) is also referred to as 15N3K herein. The composition according to the invention may be used in the therapeutic aspects of the invention described herein.

In a second aspect the present invention provides the composition of the invention as described herein for use in treating and/or preventing uveitis in a subject.

A peptide composition according to the invention comprises the amino acid sequences according to the invention as described herein. In one aspect the peptide composition comprises only the amino acid sequences according to the invention as described herein.

The subject may express HLA-DR3. The subject may express HLA-DR2.

The composition of the invention may be administered following a dose-escalation protocol.

In another aspect the present invention relates to a kit which comprises the following S-Ag peptides:
the amino acid sequence KKKAFVEQVANVVLKKK (SEQ ID NO: 1); and
the amino acid sequence KKKLTFRRDLYFSRVQVYKKK (SEQ ID NO: 2); and
the amino acid sequence KKKVIFKKISRDKSVTIYLGKKK (SEQ ID NO: 3); being suitable for simultaneous, separate or sequential administration in the prevention or treatment of uveitis.

### DESCRIPTION OF THE FIGURES

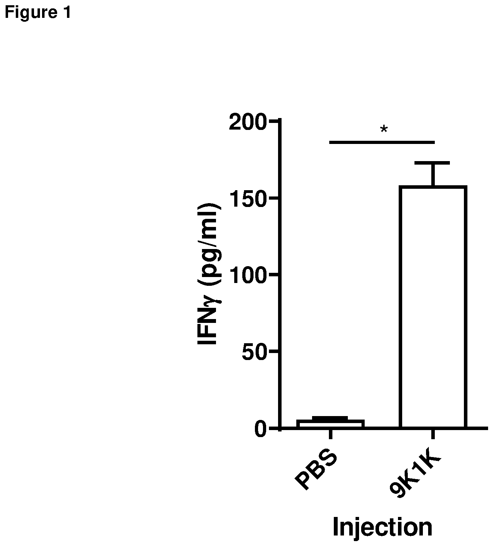

**Figure 1****:** Peptide 9K1K is correctly loaded on MHC class II in vivo. Response of peptide-specific CD4+ T cells towards peptide loaded in vivo on dendritic cells is shown. HLA-DR2-transgenic mice were injected subcutaneously with 100 µg of 9K1K peptide. 2h post injection, spleens were dissected and CD11c+ cells (dendritic cells) were isolated. These CD11c+ cells were co-cultured with peptide-specific CD4+ cells for 72h at 37°C. IFNγ was measured in the supernatant of these cultures to evaluate the response to peptide by the CD4+ T cells. (* p<0.05 Mann-Whitney U-test)

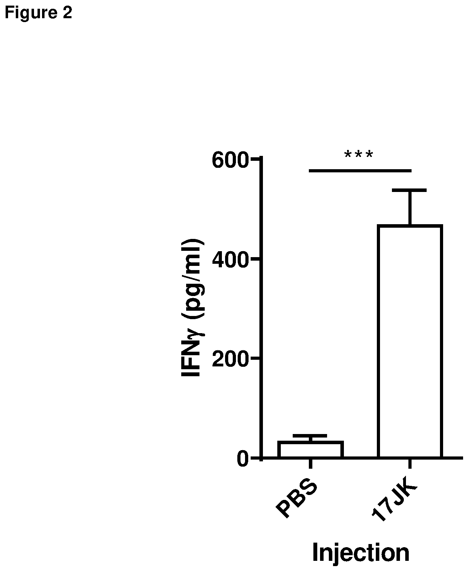

**Figure 2****:** Peptide 17JK is correctly loaded on MHC class II in vivo. Response of peptide-specific CD4+ T cells towards peptide loaded in vivo on dendritic cells is shown. HLA-DR3-transgenic mice were injected subcutaneously with 100 µg of 17JK peptide. 2h post injection, spleens were dissected and CD11c+ cells (dendritic cells) were isolated. These CD11c+ cells were co-cultured with peptide-specific CD4+ cells for 72h at 37°C. IFNγ was measured in the supernatant of these cultures to evaluate the response to peptide by the CD4+ T cells. (*** p<0.001 Mann-Whitney U-test)

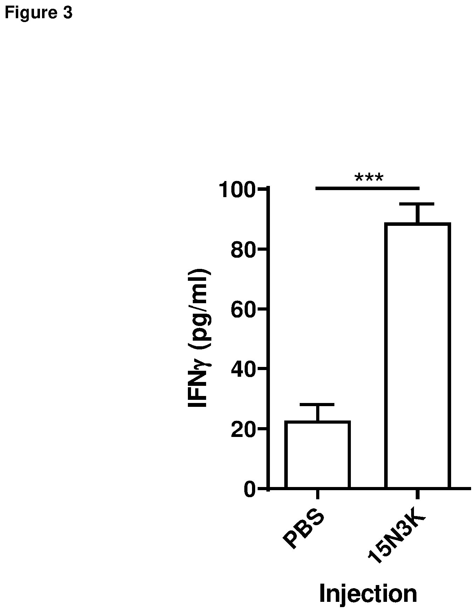

**Figure 3****:** Peptide 15N3K is correctly loaded on MHC class II in vivo. Response of peptide-specific CD4+ T cells towards peptide loaded in vivo on dendritic cells is shown. HLA-DR3-transgenic mice were injected subcutaneously with 100 µg of 15N3K peptide. 2h post injection, spleens were dissected and CD11c+ cells (dendritic cells) were isolated. These CD11c+ cells were co-cultured with peptide-specific CD4+ cells for 72h at 37°C. IFNγ was measured in the supernatant of these cultures to evaluate the response to peptide by the CD4+ T cells. (*** p<0.001 Mann-Whitney U-test)

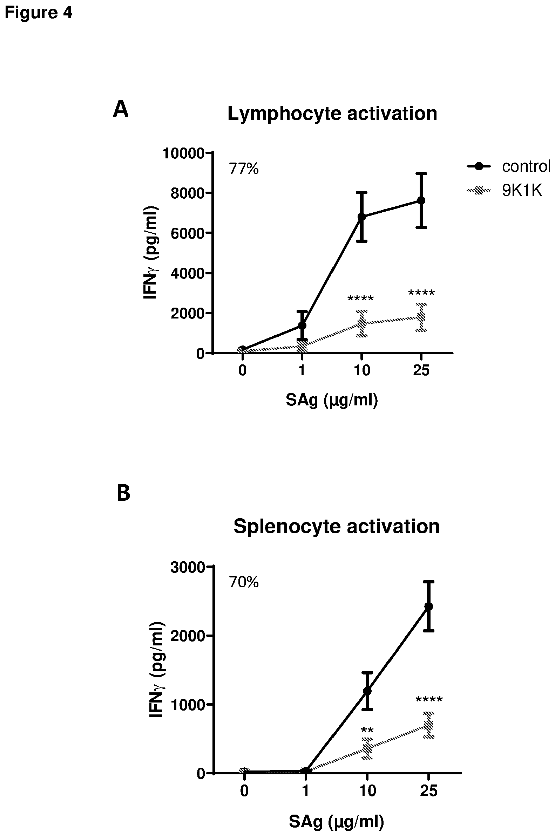

**Figure 4****:** 9K1K alone induces tolerance towards S-Ag protein in DR2tg mice. Mice were injected subcutaneously in the flank with 0,1 µg/ml, 1 µg/ml and 10 µg/ml 9K1K on days -15, -13 and -11, followed by 3 injections of 100 µg/ml on days -8, -6 and -4 (escalating dose regime). On day 0, mice were immunized subcutaneously at the base of the tail with antigen/CFA. Mice were sacrificed 10 days after immunization to measure activation of LN cells and splenocytes upon S-Ag restimulation. IFNγ concentration was measured in the culture supernatant as an indication of cell activation. Data represent mean ± SEM of the concentration of IFNγ for the PBS-treated mice (black lines) and 9K1K-treated mice (grey lines). Two-way ANOVA was used to measure overall treatment effects on T cell activation. Dunnett's multiple comparisons test was used and significant differences are indicated in the graphs (** p<0.01; **** p<0.0001). The % inhibition of IFNγ production as compared to the control group is indicated in the graphs. **(A)** Tolerization against S-Ag in LN. IFNγ production expressed as IFNγ concentration (pg/ml). **(B)** Tolerization against S-Ag in spleen. IFNγ production expressed as IFN-γ concentration (pg/ml). LN, lymph nodes. Data representative of 3 independent experiments.

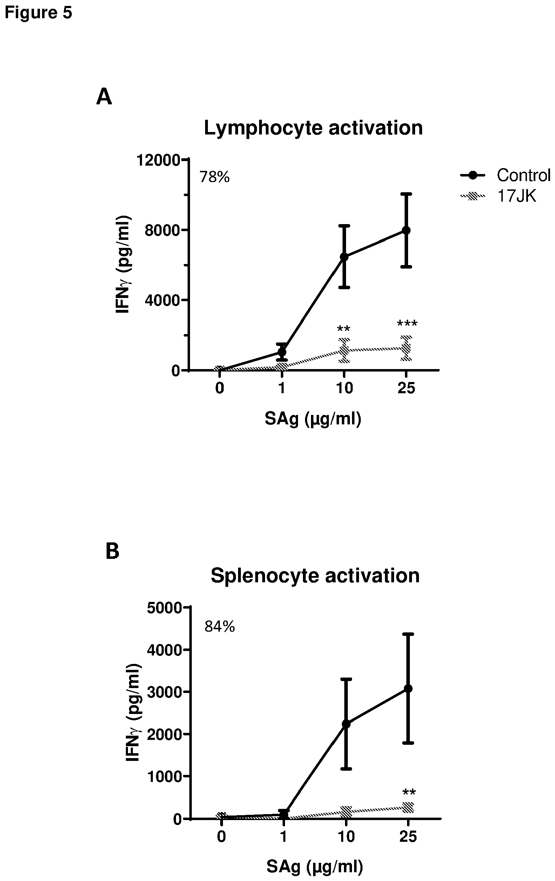

**Figure 5****:** 17JK alone induces tolerance towards S-Ag protein in DR3tg mice. Mice were injected subcutaneously in the flank with 0,1 µg/ml, 1 µg/ml and 10 µg/ml 17JK on days -15, -13 and -11, followed by 3 injections of 100 µg/ml on days -8, -6 and -4 (escalating dose regime). On day 0, mice were immunized subcutaneously at the base of the tail with antigen/CFA. Mice were sacrificed 10 days after immunization to measure activation of LN cells and splenocytes upon S-Ag restimulation. IFNγ concentration was measured in the culture supernatant as an indication of cell activation. Data represent mean ± SEM of the concentration of IFNγ for the PBS-treated mice (black lines) and 17JK-treated mice (grey lines). Two-way ANOVA was used to measure overall treatment effects on T cell activation. Dunnett's multiple comparisons test was used and significant differences are indicated in the graphs (** p<0.01; *** p<0.001). The % inhibition of IFNγ production as compared to the control group is indicated in the graphs. **(A)** Tolerization against S-Ag in LN. IFNγ production expressed as IFNγ concentration (pg/ml). **(B)** Tolerization against S-Ag in spleen. IFNγ production expressed as IFN-γ concentration (pg/ml). LN, lymph nodes. Data representative of 3 independent experiments.

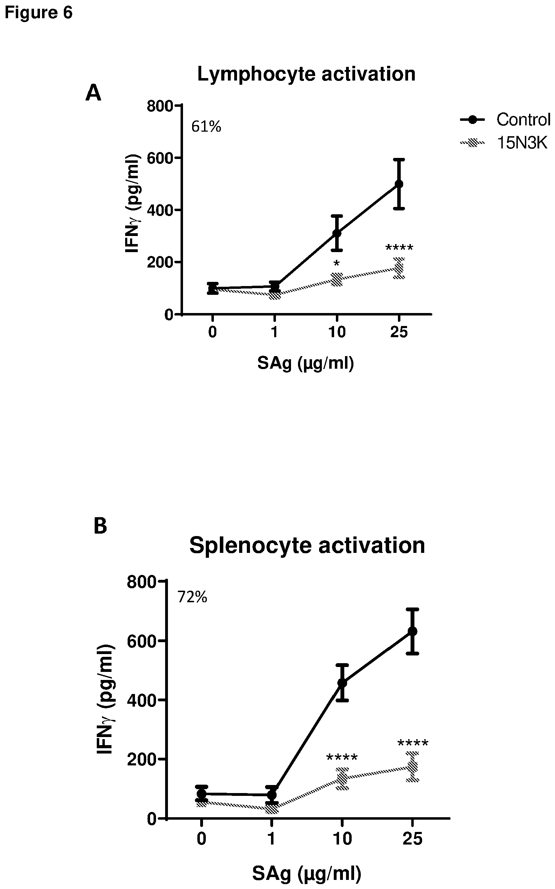

**Figure 6****:** 15N3K alone induces tolerance towards S-Ag protein in DR3tg mice. Mice were injected subcutaneously in the flank with 0,1 µg/ml, 1 µg/ml and 10 µg/ml 15N3K on days -15, -13 and -11, followed by 3 injections of 100 µg/ml on days -8, -6 and -4 (escalating dose regime). On day 0, mice were immunized subcutaneously at the base of the tail with antigen/CFA. Mice were sacrificed 10 days after immunization to measure activation of LN cells and splenocytes upon S-Ag restimulation. IFNγ concentration was measured in the culture supernatant as an indication of cell activation. Data represent mean ± SEM of the concentration of IFNγ for the PBS-treated mice (black lines) and 15N3K-treated mice (grey lines). Two-way ANOVA was used to measure overall treatment effects on T cell activation. Dunnett's multiple comparisons test was used and significant differences are indicated in the graphs (* p<0.05; **** p<0.0001). The % inhibition of IFNγ production as compared to the control group is indicated in the graphs. **(A)** Tolerization against S-Ag in LN. IFNγ production expressed as IFNγ concentration (pg/ml). **(B)** Tolerization against S-Ag in spleen. IFNγ production expressed as IFN-γ concentration (pg/ml). LN, lymph nodes. Data representative of 3 independent experiments.

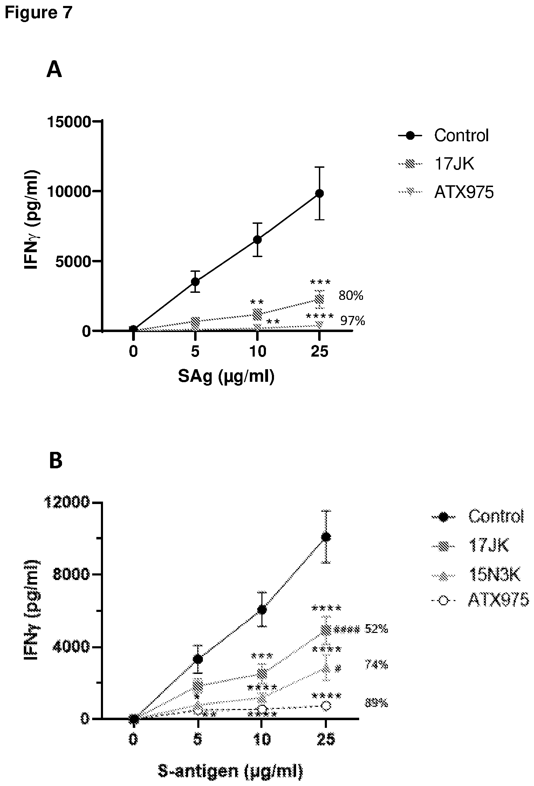

**Figure 7****:** Treatment with peptide cocktail ATX975 reduces S-Ag-induced immune cell activation in DR3tg mice more efficiently than treatment with 17JK or 15N3K alone. Mice were injected subcutaneously in the flank with 0.015 nmol, 0.15 nmol and 1.5 nmol of peptide on days -15, -13 and -11 respectively, followed by 3 injections of 15 nmol peptide on days -8, -6 and -4 (dose escalation schedule). For treatment with the cocktail (ATX975) containing 3 peptides, these doses were given per peptide (reaching a total top dose of 45 nmol peptide). On day 0, mice were immunized subcutaneously at the base of the tail with antigen/CFA. Mice were sacrificed 10 days after immunization to measure activation of splenocytes upon S-Ag restimulation. Data represent mean ± SEM of the IFNγ concentration for the control-treated mice (black lines) and peptide-treated mice (grey lines). Two-way ANOVA was used to measure overall treatment effects on T cell activation. Dunnett's multiple comparisons test was used and significant differences are indicated in the graphs (* p<0.05; ** p<0.01; *** p<0.001; **** p<0.0001 as compared to the control group; ^{#} p<0.05, ^{####} p<0.0001 as compared to the ATX975 group). The % inhibition of IFNγ production as compared to the control group is indicated in the graphs. **(A)** Tolerization against S-Ag in spleen. IFN-γ production expressed as IFN-γ concentration (pg/ml). **(B)** Tolerization against S-Ag in spleen. IFN-γ production expressed as IFN-γ concentration (pg/ml).

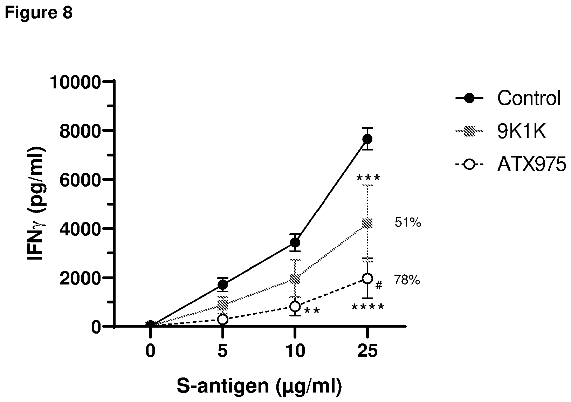

**Figure 8****:** Treatment with peptide cocktail ATX975 reduces S-Ag-induced immune cell activation in DR2tg mice more efficiently than treatment with 9K1K alone. Mice were injected subcutaneously in the flank with 0.015 nmol, 0.15 nmol and 1.5 nmol of peptide on days -15, -13 and -11 respectively, followed by 3 injections of 15 nmol peptide on days -8, -6 and -4 (dose escalation schedule). For treatment with the cocktail (ATX975) containing 3 peptides, these doses were given per peptide (reaching a total top dose of 45 nmol peptide). On day 0, mice were immunized subcutaneously at the base of the tail with antigen/CFA. Mice were sacrificed 10 days after immunization to measure activation of splenocytes upon S-Ag restimulation. Data represent mean ± SEM of the IFNγ concentration for the control-treated mice (black lines) and peptide-treated mice (grey lines). Two-way ANOVA was used to measure overall treatment effects on T cell activation. Dunnett's multiple comparisons test was used and significant differences are indicated in the graphs (* p<0.05; ** p<0.01). The % inhibition of IFNγ production as compared to the control group is indicated in the graphs. Tolerization against S-Ag in spleen. IFN-γ production expressed as IFN-γ concentration (pg/ml).

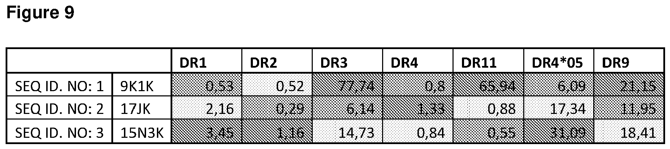

**Figure 9****:** Diverse patterns of *in vitro* peptide-MHC II binding for peptides 9K1, 17JK and 15N3K. Binding of peptides 9K1, 17JK and 15N3K to recombinant HLA-DRA1*0101, DRB1*0101 (DR1), DRB1*1501 (DR2), DRB1*0301 (DR3), DRB1*0401 (DR4), DRB1*1101 (DR11), DRB1*0405 (DR4*05) and DRB1*0901 (DR9) was assessed *in vitro.* IC50 values (µM) are presented and colour-coded per HLA-DR molecule. Low values (green) indicate strong binding, high values (red) indicate low(er) binding.

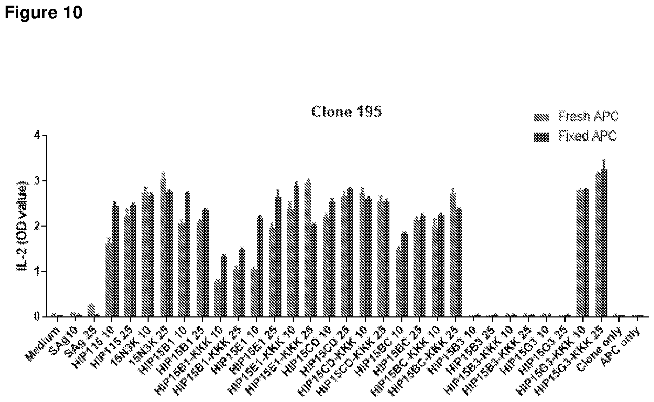

**Figure 10****:** Variants of peptide 15N3K are apitopes. The ability of variants of the 15N3K peptide to behave as apitopes (i.e. bind to an MHCII molecule and be presented to a T cell by an antigen-presenting cell without undergoing antigen processing) was tested using an APIPS assay. DR3tg mice were immunised with SAg and hybridoma's were generated. 5*10⁴ SAg-specific hybridoma cells were cultured with 5*10⁴ fresh or fixed commercial APC (VAVY) cells. Cultures were stimulated with 10 or 25 µg/ml of peptide, as indicated in the graph. T cell activation was measured by IL-2 ELISA on supernatants collected after 48h. The graph represents the mean of duplicate measurements ± SEM.

### DETAILED DESCRIPTION OF THE INVENTION

The present invention provides a new and alternative therapeutic option for the treatment and/or prevention of uveitis. As demonstrated in the present application, a combination of peptides of SEQ ID NOs: 1, 2 and 3 induces tolerance towards S-Ag in a tolerance model in HLA-DR transgenic mice.

The present invention is defined by the claims.

As such, the first aspect of the invention relates to a composition which comprises a plurality of peptides from S-Ag, namely peptides of the invention as defined herein, namely peptides of SEQ ID NO: 1, 2 and 3.

### S-arrestin

S-arrestin (also known as retinal arrestin, S-antigen or S-Ag) is a soluble photoreceptor protein expressed in the retina and the pineal gland. It is known to be involved in desensitization of the photoactivated transduction cascade and was first isolated from its binding to activated rhodopsin. The crystal structure shows two domains of anti-parallel β-sheets joined by a hinge region as well as a short α-helix at the back of the amino terminal fold.

The light-activated form of the visual pigment rhodopsin (Rh*) interacts with the retinal G protein transducin, thereby initiating the exchange of a GDP molecule for GTP at the alpha-subunit of transducin. In its GTP-binding form transducin dissociates from Rh*, and activates a cyclic GMP phosphodiesterase (PDE), by binding to its two inhibitory subunits PDEγ. The result is a rapid decline in the concentration of the internal transmitter cyclic GMP. Because the interaction of Rh* and transducin takes only about 1 ms, a single Rh* can subsequently interact with hundreds of transducin molecules. The turnover number for PDE can be in the order of several thousand hydrolysed cGMP per PDE per second. Thus, for a limitation of the light response as well as for a fast recovery, a rapid and effective elimination of Rh* is essential, before it can activate too many PDE molecules. This inactivation of Rh* is accomplished in two steps: phosphorylation of Rh* reduces its ability to catalyse the nucleotide exchange of transducin and subsequent binding of arrestin to P-Rh* completely shields it from further interaction with transducin.

The amino acid sequence of mature human S-Ag is given below (SEQ ID NO: 4).

### UniProt database P10523 (https://www.uniprot.org/)

10 20 30 40 50
MAASGKTSKS EPNHVIFKKI SRDKSVTIYL GNRDYIDHVS QVQPVDGVVL
60 70 80 90 100
VDPDLVKGKK VYVTLTCAFR YGQEDIDVIG LTFRRDLYFS RVQVYPPVGA
110 120 130 140 150
ASTPTKLQES LLKKLGSNTY PFLLTFPDYL PCSVMLQPAP QDSGKSCGVD
160 170 180 190 200
FEVKAFATDS TDAEEDKIPK KSSVRLLIRK VQHAPLEMGP QPRAEAAWQF
210 220 230 240 250
FMSDKPLHLA VSLNKEIYFH GEPIPVTVTV TNNTEKTVKK IKAFVEQVAN
260 270 280 290 300
VVLYSSDYYV KPVAMEEAQE KVPPNSTLTK TLTLLPLLAN NRERRGIALD
310 320 330 340 350
GKIKHEDTNL ASSTIIKEGI DRTVLGILVS YQIKVKLTVS GFLGELTSSE
360 370 380 390 400
VATEVPFRLM HPQPEDPAKE SYQDANLVFE EFARHNLKDA GEAEEGKRDK

### NDVDE

### Uveitis

Clinically, uveitis is commonly classified as one of the following based on the part of the eye which is primarily affected: anterior uveitis, intermediate uveitis, posterior uveitis or panuveitis.

Anterior uveitis is the most common form of uveitis and includes iridocyclitis and iritis. Iritis is the inflammation of the anterior chamber and iris, while iridocyclitis includes inflammation in the ciliary body.

Intermediate uveitis (pars planitis) commonly refers to vitritis - inflammation of cells in the vitreous cavity, associated with deposition of inflammatory material on the pars plana.

Posterior uveitis (chorioretinitis) is the inflammation of the retina and choroid regions.

Panuveitis uveitis is a general term referring to inflammation affecting all layers of the uvea.

Uveitis can also be classified as either infectious or non-infectious, with uveitis related to autoimmune diseases (i.e. primarily non-infectious) being more common in developed countries. The common animal models used to study uveitis are also driven by autoimmunity, showing a clear association between the two. It is predicted that 25-30% of uveitis is associated with systemic autoimmune or autoinflammatory diseases.

In one aspect of the invention the uveitis is non-infectious uveitis.

### Tolerance

T cell epitopes play a central role in the adaptive immune response to any antigen, whether self or foreign. The central role played by T cell epitopes in hypersensitivity diseases (which include allergy and transplant rejection) has been demonstrated through the use of experimental models. It is possible to induce autoimmune or allergic diseases by injection of synthetic peptides (based on the structure of T cell epitopes) in combination with adjuvant.

By contrast, it has been shown to be possible to induce immunogenic tolerance towards particular antigens by administration of peptide epitopes in soluble form. Administration of soluble peptide has been demonstrated as an effective means of inhibiting disease in experimental autoimmune encephalomyelitis (EAE - a model for multiple sclerosis (MS)) (Metzler and Wraith (1993) Int. Immunol. 5:1159-1165; Liu and Wraith (1995) Int. Immunol. 7:1255-1263; Anderton and Wraith (1998) Eur. J. Immunol. 28:1251-1261); and experimental models of arthritis, diabetes, and uveoretinitis (reviewed in Anderton and Wraith (1998) as above). This has also been demonstrated as a means of treating an ongoing disease in EAE (Anderton and Wraith (1998) as above).

Tolerance is the failure to respond to an antigen. Tolerance to self antigens is an essential feature of the immune system, when this is lost, autoimmune disease can result. The adaptive immune system must maintain the capacity to respond to an enormous variety of infectious agents while avoiding autoimmune attack of the self antigens contained within its own tissues. This is controlled to a large extent by negative selection of high-affinity T lymphocytes in the thymus (central tolerance). However, not all self antigens are expressed in the thymus, so death of self-reactive thymocytes remains incomplete. There are thus also mechanisms by which tolerance may be acquired by mature self-reactive T lymphocytes in the peripheral tissues (peripheral tolerance). A review of the mechanisms of central and peripheral tolerance is given in Anderton et al (1999) Immunological Reviews 169:123-137. See also Wraith (2016) Nature 530:422-423.

Available data suggests that uveitis can result from autoreactive T cells being generated from retinal proteins, including S-Ag, driving inflammation and causing chronic disease. The composition of the present invention is capable of inducing tolerance to self-antigens such as S-Ag, such that when administered to a subject, it may reinstate tolerance to the S-Ag protein and curtail the pathogenic immune response.

### Apitopes

In an adaptive immune response, T lymphocytes are capable of recognising epitopes of a protein antigen. APCs take up protein antigens and degrade them into short peptide fragments. A peptide may bind to a major histocompatibility complex (MHC) inside the cell and be carried to the cell surface. When presented at the cell surface in conjunction with an MHC molecule, the peptide may be recognised by a T cell (via the T cell receptor (TCR), in which case the peptide is a T cell epitope.

An epitope is thus a peptide derivable from an antigen which is capable of binding to the peptide-binding groove of an MHC molecule and being recognised by a T cell.

The minimal epitope is the shortest fragment derivable from an epitope, which is capable of binding to the peptide-binding grove of an MHC class I or II molecule and being recognised by a T cell. For a given immunogenic region, it is typically possible to generate a "nested set" of overlapping peptides which act as epitopes, all of which contain the minimal epitope but differ in their flanking regions.

By the same token, it is possible to identify the minimal epitope for a particular MHC molecule: T cell combination by measuring the response to truncated peptides. For example, if a response is obtained to the peptide comprising residues 1-15 in the overlapping library, sets which are truncated at both ends (i.e. 1-14, 1-13, 1-12 etc. and 2-15, 3-15, 4-15 etc.) can be used to identify the minimal epitope.

The present inventors have previously determined that there is a link between the capacity of a peptide to bind to an MHC molecule and be presented to a T cell without further processing, and the peptide's capacity to induce tolerance in vivo (WO 02/16410). If a peptide is too long to bind the peptide binding groove of an MHC molecule without further processing (e.g. trimming), or binds in an inappropriate conformation then it will not be tolerogenic in vivo. If, on the other hand, the peptide is of an appropriate size and conformation to bind directly to the MHC peptide binding groove and be presented to a T cell, then this peptide can be predicted to be useful for tolerance induction.

It is thus possible to investigate the tolerogenic capacity of a peptide by investigating whether it can bind to an MHC molecule and be presented to a T cell without further antigen processing in vitro.

S-Ag apitopes (Antigen Processing-Independent epiTOPES) are capable of binding to an MHC class II molecule and stimulating a response from S-Ag specific T cells without further antigen processing. Such apitopes can be predicted to cause tolerance to SAg, following the rule-based method described in WO 02/16410.

Peptides that bind to MHC class I molecules are typically 7 to 13, more usually 8 to 10 amino acids in length. The binding of the peptide is stabilised at its two ends by contacts between atoms in the main chain of the peptide and invariant sites in the peptide-binding groove of all MHC class I molecules. There are invariant sites at both ends of the groove which bind the amino and carboxy termini of the peptide. Variations in peptide length are accommodated by a kinking in the peptide backbone, often at proline or glycine residues that allow flexibility.

Peptides which bind to MHC class II molecules are typically between 8 and 20 amino acids in length, more usually between 10 and 17 amino acids in length and can be longer (for example up to 40 amino acids). These peptides lie in an extended conformation along the MHC II peptide-binding groove which (unlike the MHC class I peptide-binding groove) is open at both ends. The peptide is held in place mainly by main-chain atom contacts with conserved residues that line the peptide-binding groove.

In a preferred embodiment, the peptide derived from S-Ag is capable of binding to an MHC class II molecule without further processing.

### Peptides

The term "peptide" is used in the normal sense to mean a series of residues, typically L-amino acids, connected one to the other, typically by peptide bonds between the a-amino and carboxyl groups of adjacent amino acids. The term includes modified peptides and synthetic peptide analogues.

The peptide of the present invention may be made using chemical methods (Peptide Chemistry, A practical Textbook. Mikos Bodansky, Springer-Verlag, Berlin.). For example, peptides can be synthesized by solid phase techniques (Roberge JY et al (1995) Science 269: 202-204), cleaved from the resin, and purified by preparative high performance liquid chromatography (e.g., Creighton (1983) Proteins Structures And Molecular Principles, WH Freeman and Co, New York NY). Automated synthesis may be achieved, for example, using the ABI 43 1 A Peptide Synthesizer (Perkin Elmer) in accordance with the instructions provided by the manufacturer.

The peptide may alternatively be made by recombinant means, or by cleavage from a longer polypeptide. For example, the peptide may be obtained by cleavage from the S-antigen protein, which may be followed by modification of one or both ends. The composition of a peptide may be confirmed by amino acid analysis or sequencing (e.g. the Edman degradation procedure).

For practical purposes, there are various other characteristics which the peptide may show. For example, it is important that the peptide is sufficiently stable in vivo to be therapeutically useful. The half-life of the peptide *in vivo* may be at least 10 minutes, 30 minutes, 4 hours, or 24 hours.

The peptides used in the present invention are as follows:
a peptide consisting of the amino acid sequence KKKAFVEQVANWLKKK (SEQ ID NO: 1); and
a peptide consisting of the amino acid sequence KKKLTFRRDLYFSRVQVYKKK (SEQ ID NO: 2); and
a peptide consisting of the amino acid sequence KKKVIFKKISRDKSVTIYLGKKK (SEQ ID NO: 3).

Sequence identity may be assessed by any convenient method. However, for determining the degree of sequence identity between sequences, computer programs that make multiple alignments of sequences are useful, for instance Clustal W (Thompson et al., (1994) Nucleic Acids Res., 22: 4673-4680). Programs that compare and align pairs of sequences, like ALIGN (Myers et al., (1988) CABIOS, 4: 1-17), FASTA (Pearson et al., (1988) PNAS, 85:2444-2448; Pearson (1990), Methods Enzymol., 183: 63-98) and gapped BLAST (Altschul et al., (1997) Nucleic Acids Res., 25: 3389-3402) are also useful for this purpose. Furthermore, the Dali server at the European Bioinformatics institute offers structure-based alignments of protein sequences (Holm (1993) J. Mol. Biol., 233: 123-38; Holm (1995) Trends Biochem. Sci., 20: 478-480; Holm (1998) Nucleic Acid Res., 26: 316-9).

Multiple sequence alignments and percent identity calculations may be determined using the standard BLAST parameters, (using sequences from all organisms available, matrix Blosum 62, gap costs: existence 11, extension 1).

Alternatively, the following program and parameters may be used: Program: Align Plus 4, version 4.10 (Sci Ed Central Clone Manager Professional Suite). DNA comparison: Global comparison, Standard Linear Scoring matrix, Mismatch penalty = 2, Open gap penalty = 4, Extend gap penalty = 1. Amino acid comparison: Global comparison, BLOSUM 62 Scoring matrix.

Thus described herein are variants of the stated or given sequences, as long as the variant retains the functional activity of the parent i.e. the variants are functionally equivalent, in other words they have or exhibit an activity of the parent peptide as defined herein. Such variants may comprise amino acid substitutions, additions or deletions (including truncations at one or both ends) of the parent sequence e.g. of one or more e.g. 1 to 14 amino acids.

Also included are functionally-equivalent derivatives in which one or more of the amino acids are chemically derivatised, e.g. substituted with a chemical group.

The peptide may comprise between 8 and 30 amino acids, for example 8 to 25 amino acids, 8 to 20 amino acids, 8 to 15 amino acids or 8 to 12 amino acids. In one aspect the peptide may thus be 8, 9, 10, 11, 12, 13, 14, 15, 16, 17, 18, 19, 20, 21, 22, 23, 24, 25, 26, 27, 28, 29 or 30 amino acids in length.

The peptide is an apitope, i.e. capable of binding to an MHC molecule *in vitro and in vivo* and presented to a T cell without antigen processing.

### Solubility

Solubility may be an important consideration in peptide-mediated tolerance induction.

Solubility may be improved by incorporation of additional amino acids which may be Glycine (G), Lysine (K) and/or Glutamic acid (E) at both N and C termini.

A peptide may have, for example, one, two or three additional amino acids at the N and/or C termini. The additional amino acids may be selected from Glycine (G), Lysine (K) and/or Glutamic acid (E). Different combinations of these amino acids may be added to the peptides.

For example, a peptide may have one, two or three Lysine (K) residues at both N and C termini.

A peptide may have one, two or three Glycine (G) residues at both N and C termini.

A peptide may have one, two or three Glutamic acid (E) residues at both N and C termini.

A peptide may have one Glycine and one Lysine residue at both N and C termini.

A peptide may have one Glycine and two Lysine residues at both N and C termini.

A peptide may have one Glutamic acid and one Lysine residue at both N and C termini.

A peptide may have one Glutamic acid and two Lysine residues at both N and C termini.

For example, the additional amino acids may comprise a glycine or lysine spacer, followed by the amino acid pairs KK, KE, EK or EE at one or both ends.

The peptide may have a Glycine spacer at both ends, followed by combinations of two additional amino acids which may be Lysine (K) and/or Glutamic acid (E) at both N and C termini. The possible combination at a given terminus may therefore be GKK, GKE, GEK or GEE.

The peptide may have the general formula:
XXG - parent peptide - GXX

A peptide may have three additional Lysine (K) residues at both N and C termini.

Modified peptides may therefore have 6 additional amino acids (3 at each end) than the parent peptides.

The peptides may alternatively have the general formula:
KKK - parent peptide - KKK
KK - parent peptide - KK
K - parent peptide - K
GK - parent peptide - KG
GKK - parent peptide - KKG
KKG-parental peptide-GKK
EK - parent peptide - KE
EKK - parent peptide - KKE
GKE - parent peptide - EKG
GEK - parent peptide - KEG

The modified peptide may be more soluble that the parent (unmodified) peptide. The modified peptide may have 2, 3, 4, or 5-fold greater solubility than the parent peptide. The peptide may be soluble at concentrations of up to 0.5 mg/ml, 1 mg/ml, or 5 mg/ml. As discussed herein, the modified peptides may have 2, 4 or 6 additional amino acids (1, 2 or 3 at each end) than the parent peptides.

In the most preferred aspect, the modification is the inclusion of KKK at both the N and C termini.

The modified peptide may be more soluble than the parent (unmodified) peptide. The modified peptide may have 2, 3, 4, or 5-fold greater solubility than the parent peptide. The peptide may be soluble at concentrations of up to 0.5 mg/ml, 1 mg/ml, 5 mg/ml or higher, for example 8 mg/ml. The modified peptide may be soluble at a concentration of 4mg/ml.

### Composition

The S-Ag peptides are in the form of a composition, preferably a pharmaceutical composition.

A peptide composition according to the invention comprises the amino acid sequences according to the invention as described herein. In one aspect the peptide composition comprises only the amino acid sequences according to the invention as described herein, i.e. it does not comprise additional peptides other than those according to the invention.

In one aspect of the disclosure not forming a part of the invention discussed herein, as a first step a subject is identified who has or is at risk of developing uveitis.

The peptides or composition according to the present invention may be for prophylactic or therapeutic use.

When administered for prophylactic use, the peptides or composition may reduce or prevent the generation of an immune response to S-Ag. The level of immune response is less than would be obtained if the subject had not been treated with the composition.

The term "reduce" indicates that a partial reduction in immune response is observed, such as a 50%, 60%, 70%, 80%, or 90% reduction in the response that would have been observed if the subject had not been treated with the composition (or in response observed in an untreated subject over the same time-period). The term "prevent" indicates that no appreciable immune response to S-Ag is observed.

When administered for therapeutic use, the peptides or composition may suppress an already on-going immune response to S-Ag. The term "suppress" indicates a reduction in the level of an on-going immune response, compared to the level before peptide treatment or the levels which would have been observed at the same time point had the treatment not been given.

Treatment with the composition of the present invention may cause a reduction in level of any or all of the following:
i) S-Ag autoantibodies
ii) proinflammatory CD4+ T cells specific for S-Ag
iii) B cells secreting S-Ag autoantibodies.

Detection of all of the factors can be carried out by techniques known in the art, such as ELISA, flow cytometry etc.

Treatment with the composition of the present invention may also or alternatively cause anergy in CD4+ T cells specific for S-Ag. Anergy can be detected by, for example subsequent challenge with S-Ag in vitro.

### Formulation

The composition may be a pharmaceutical composition which additionally comprises a pharmaceutically acceptable carrier, diluent or excipient. The pharmaceutical composition may optionally comprise one or more further pharmaceutically active compounds. Such a formulation may, for example, be in a form suitable for intradermal or subcutaneous administration

The composition may by prepared as an injectable, either as liquid solution or suspension; solid form suitable for solution in, or suspension in, liquid prior to injection may also be prepared. The peptide may alternatively be encapsulated in a carrier or bound to the surface of a carrier, for example a nanoparticle. The active ingredients may be mixed with excipients which are pharmaceutically acceptable and compatible with the active ingredient. Suitable excipients are, for example, water, saline (for example, phosphate-buffered saline), dextrose, glycerol, ethanol, or the like and combinations thereof.

In addition, if desired, the composition may contain minor amounts of auxiliary substances such as wetting or emulsifying agents and/or pH buffering agents. Buffering salts include phosphate, citrate, acetate. Hydrochloric acid and/or sodium hydroxide may be used for pH adjustment. For stabilisation, disaccharides may be used such as sucrose or trehalose.

In the composition, the relative ratio of the peptides may be approximately 1:1:1. Alternatively the relative ratios of each peptide may be altered, for example, if it is found that one peptide works better than the others in particular HLA types.

After formulation, the composition may be incorporated into a sterile container which is then sealed and stored at a low temperature, for example 4°C, or it may be freeze-dried.

Conveniently the composition is prepared as a lyophilised (freeze-dried) powder. Lyophilisation permits long-term storage in a stabilised form. Lyophilisation procedures are well known in the art, see for example http://www.devicelink.com/ivdt/archive/97/01/006.html. Bulking agents are commonly used prior to freeze-drying, such as mannitol, dextran or glycine.

The composition may be administered in a convenient manner such as by the oral, intravenous, intramuscular, subcutaneous, sublingual, intranasal, intradermal or suppository routes or implanting (e.g. using slow release molecules).

The composition may advantageously be administered via intranasal, subcutaneous or intradermal routes. In one aspect administration may be via transdermal patches.

The peptide or composition as described herein is typically administered in an "effective amount"; that is, an amount effective to elicit any one or more inter alia of a therapeutic or prophylactic effect. Persons skilled in the art would be able, by routine experimentation, to determine an effective, non-toxic amount to include in a pharmaceutical composition or to be administered for the desired outcome. In general, the peptide or composition as disclosed herein can be administered in a manner compatible with the route of administration and physical characteristics of the recipient (including health status) and in such a way that it elicits the desired effect(s) (i.e. therapeutically effective and/or protective). For example, the appropriate dosage of a composition may depend on a variety of factors including, but not limited to, a subject's physical characteristics (e.g., age, weight, sex), and other factors that may be recognized by persons skilled in the art. Other illustrative examples of general considerations that may be considered when determining, for example, an appropriate dosage of the compositions are discussed by Gennaro (2000, "Remington: The Science and Practice of Pharmacy", 20th edition, Lippincott, Williams, & Wilkins; and Gilman et al., (Eds), (1990), "Goodman And Gilman's: The Pharmacological Bases of Therapeutics", Pergamon Press).

The peptide and composition of the invention may be used to treat a human subject. The subject may have uveitis. The subject may have S-Ag autoreactive T cells.

The subject may be expressing an HLA-haplotype which is associated with a predisposition to produce excessive T cells specific for S-Ag. Methods for determining the HLA haplotype of an individual are known in the art. In one aspect the subject has an HLA gene selected from the following: A29, B51, B27, DR8, DR4, DP5, DR4, DQA3, DR3, DR2, DR51, and DR17 (see Mattapallil et al. J Immunol 2011, 187:1977-1985).

### Dose escalation protocol

In a preferred embodiment the peptides or composition of the invention may be administered to the subject using a "dose escalation" protocol, where a plurality of doses is given to the subject in ascending concentrations. Such an approach has been used, for example, for phospholipase A2 peptides in immunotherapeutic applications against bee venom allergy (Müller et al (1998) J. Allergy Clin Immunol. 101:747-754 and Akdis et al (1998) J. Clin. Invest. 102:98-106).

In one aspect the dose escalation protocol may comprise administering to the subject a tolerogenic peptide in the following doses:
Day 1: a first dose of about 15 to about 40µg;
Day 14±7 days: a second dose of about 35-65µg;
Day 28±7 days: a third dose of about 80-120µg;
Day 42±7 days : a fourth dose of about 300-500µg;
Day 56±7 days: a fifth dose of about 300-1800µg;
Day 70±7 days: a sixth dose of about 300-1800µg;
Day 84±7 days: a seventh dose of about 300-1800µg;
Day 98±7 days: an eighth dose of about 300-1800µg;
Day 112±7 days: a ninth dose of about 300-1800µg; and
Day 126±7 days: a tenth dose of about 300-1800µg.

In one aspect of the invention as described herein the fifth to tenth doses may be about 600 -1500 µg.

The reference to "±7 days" is intended to refer to the stated day (i.e. the stated day following the first administration of the peptide, which is taken as day 1) wherein the administration can occur either up to and including seven days before, or up to and including seven days after, the stated day. As such, the administration may take place 7, 6, 5, 4, 3, 2, or 1 days before, or 1, 2, 3, 4, 5, 6 or 7 days after the stated day.

In one aspect of the invention described herein the reference to "±7 days" is preferably ±3 days. That is to say the administration may take place either up to and including three days before, or up to and including three days after the stated day. As such, the administration may take place 3, 2, or 1 days before, or 1, 2, or 3 days after the stated day.

The peptide may be administered in the following doses:
Day 1: a first dose of about 25µg;
Day 14: a second dose of about 50µg;
Day 28: a third dose of about 100µg;
Day 42: a fourth dose of about 400µg;
Day 56: a fifth dose of about 800 µg;
Day 70: a sixth dose of about 800 µg;
Day 84: a seventh dose of about 800 µg;
Day 98: a eighth dose of about 800 µg;
Day 112: a ninth dose of about 800 µg; and
Day 126: a tenth dose of about 800 µg.

In one aspect the fifth to tenth doses may alternatively be about 400µg.

In an alternative aspect the fifth to tenth doses may alternatively be about 1600µg.

As such, the peptide may be administered peptide in the following doses:
Day 1: a first dose of about 25µg;
Day 14: a second dose of about 50µg;
Day 28: a third dose of about 100µg;
Day 42: a fourth dose of about 400µg;
Day 56: a fifth dose of about 400 µg;
Day 70: a sixth dose of about 400 µg;
Day 84: a seventh dose of about 400 µg;
Day 98: a eighth dose of about 400 µg;
Day 112: a ninth dose of about 400 µg; and
Day 126: a tenth dose of about 400 µg.

Alternatively, the peptide may be administered in the following doses:
Day 1: a first dose of about 25µg;
Day 14: a second dose of about 50µg;
Day 28: a third dose of about 100µg;
Day 42: a fourth dose of about 400µg;
Day 56: a fifth dose of about 1600 µg;
Day 70: a sixth dose of about 1600 µg;
Day 84: a seventh dose of about 1600 µg;
Day 98: a eighth dose of about 1600 µg;
Day 112: a ninth dose of about 1600 µg; and
Day 126: a tenth dose of about 1600 µg.

In a further aspect of the invention as described herein an eleventh dose of about 300-1800 µg, preferably about 600-1500µg, preferably about 1200µg, more preferably about 400, 800 or 1600 µg, is administered on day 140 ±7 days, preferably on day 140±3 days, more preferably on day 140. In a preferred aspect the dose is about 800 µg.

In an even more preferred aspect, a twelfth dose of about 300-1800 µg, preferably about 600-1500µg, preferably about 1200µg, more preferably about 400, 800 or 1600µg, is administered on day 154±7 days, preferably on day 154±3 days, more preferably on day 154. In a preferred aspect the dose is about 800 µg.

In a further preferred aspect, a thirteenth dose of about 300-1800 µg, preferably about 600-1500µg, preferably about 1200µg, more preferably about 400, 800 or 1600µg, is administered on day 168±7 days, preferably on day 168±3 days, more preferably on day 168. In a preferred aspect the dose is about 800 µg.

In a further preferred aspect, a fourteenth dose of about 300-1800 µg, preferably about 600-1500µg, preferably about 1200µg, more preferably about 400, 800 or 1600µg, is administered on day 182±7 days, preferably on day 182±3 days, more preferably on day 182. In a preferred aspect the dose is about 800 µg.

Additional doses as described above may be administered as required for a period of, for example, one month to twenty years, for example for a period of one month, two months, three months, four months, five months, six months, seven months, eight months, nine months, ten months, eleven months, one year, two years, three years, four years, five years, six years, seven years, eight years, nine years, ten years, eleven years, twelve years, thirteen years, fourteen years, fifteen years, sixteen years, seventeen years, eighteen years, nineteen years or twenty years.

Any of the teachings herein regarding the "method" of the invention are equally applicable to the "uses" encompassed by the invention.

### Kit

Peptides derived from S-Ag may be administered together, in the form of a mixed composition or cocktail. However, there may be circumstances in which it is preferable to provide the peptides separately in the form of a kit, for simultaneous, separate, sequential or combined administration.

For example, the kit may comprise the peptides in separate containers. The contents of the containers may or may not be combined prior to administration.

The kit may also comprise mixing and/or administration means (for example a vapouriser for intranasal administration; or a syringe and needle or other medical device for subcutaneous/intradermal dosing). The kit may also comprise instructions for use.

The pharmaceutical composition or kit of the invention may be used to treat and/or prevent a disease, such as uveitis as discussed herein.

In particular, the composition/kit may be used to suppress or prevent the production of S-Ag-specific CD4+ T cells (or S-Ag autoantibodies) in vivo. The composition/kit may be used to treat and/or prevent uveitis in a subject.

### EXAMPLES

### Example 1 - Peptides 9K1K, 17JK and 15N3K are correctly loaded on MHC II molecules on dendritic cells in vivo

When 100 µg of peptide 9K1K was injected in DR2tg mice or when peptide 17JK or 15N3K was injected in DR3tg mice, these peptides could be detected on dendritic cells (CD11c+ cells) in the spleens of these mice 2h post injection. Activation (IFNγ production) of peptide-specific CD4+ T cells by the isolated CD11c+ cells was used as a read-out.

In Figure 1, Figure 2 and Figure 3 it can clearly be appreciated that CD11c+ cells isolated from spleen of mice injected with 9K1K, 17JK or 15N3K respectively, induce a significant CD4+ T cell activation as compared to CD11c+ cells from spleens of PBS-injected control mice. These results demonstrate that peptides 9K1K, 17JK and 15N3K reach dendritic cells in secondary lymph organs. Furthermore, these peptides bind to the MHC II molecules on these dendritic cells in the correct conformation. As a consequence, these peptides can induce tolerance.

### Example 2 - Single peptide treatment induces S-Ag-specific T cell tolerance

We previously demonstrated the tolerogenic effects of the single peptides 9K1K, 17JK and 15N3K and these results were confirmed in these experiments. HLA-DR transgenic mice were treated according to the dose-escalation schedule with one of the peptides alone.

In the first experiment (Figure 4), DR2tg mice received 9K1K peptide or PBS as described in the methods section. Pre-treatment with this modified apitope reduced SAg-induced cell activation by 77% and 70% in the lymph nodes (Figure 4A) and spleen (Figure 4B) respectively.

To confirm the tolerogenic capacity of 17JK, DR3tg mice were treated with this modified apitope or PBS. This experiment demonstrated that pre-treatment with 17JK reduced S-Ag-induced cell activation by 78% and 84% in lymph nodes (Figure 5A) and spleen (Figure 5B) respectively .

Pre-treatment of DR3tg mice with peptide 15N3K resulted in a reduction of the S-Ag-induced cell activation by 61% and 72% in lymph nodes (Figure 6A) and spleen (Figure 6B) respectively, as compared to PBS-treated control mice.

### Example 3 - Combined peptide treatment induces S-Ag-specific T cell tolerance

The findings that a peptide treatment with individual peptides 9K1K, 17JK or 15N3K reduces the S-Ag-specific immune activation in DRtg mice, led to the investigation whether a combined treatment, administering the peptides as a cocktail, could reduce the S-Ag-specific response as well. DR3tg and DR2tg mice were treated with ATX975 according to the dose-escalation schedule and immunised with an emulsion containing all three antigens in CFA. Results are shown in Figure 7 and 8 for DR3tg and DR2tg mice respectively.

Upon ATX975 treatment in DR3tg mice (Figure 7), S-Ag-induced cell activation was reduced by 95% and 89% in spleens in two independent experiments. The tolerance induction by ATX975 was much more pronounced as compared to the tolerance induction by treatment with peptide 17JK alone (80% and 52% reduction of S-Ag-induced cell activation in spleens in two independent experiments) or peptide 15N3K alone (74% reduction of S-Ag-induced cell activation in spleens).

When DR2tg mice were treated with ATX975 (Figure 8), S-Ag-induced cell activation was reduced by 78% in the spleens of treated mice as compare to control mice. This tolerance induction was more pronounced than the 51% reduction of S-Ag-induced cell activation as seen after treatment with 9K1K alone.

These data clearly indicate an additive effect of the single peptides when combined into a peptide cocktail treatment in DRtg mice.

### Example 4 - In an in vitro peptide-MHC II binding assay, peptides 9K1K, 17JK and 15N3K demonstrate diverse binding patterns

Figure 9 depicts IC50 (µM) values of peptides 9K1K, 17JK and 15N3K binding to the indicated HLA-DR molecules and competing with a known competitor peptide. Values should be compared only within each HLA-DR molecule. Low IC50 values (green) indicate the strongest binder per HLA-DR molecule. Highest values per HLA-DR molecule (red) are indicative of the lowest binder for that HLA-DR molecule. Intermediate values are indicated in orange. These results demonstrate that the 3 peptides 9K1K, 17JK and 15N3K have different binding profiles to the investigated HLA-DR molecules. So, combining the peptides into a cocktail treatment, makes the treatment less restricted to a certain HLA-DR type.

### Conclusions

We have identified the peptide cocktail ATX975 which may contain 3 peptides from the S-Ag protein and is able to induce tolerance towards S-Ag in HLA-DR transgenic mice.

### Example 5 - Variants of peptide 15N3K behave as apitopes

Apitopes (Antigen Processing-Independent epiTOPES) are capable of binding to an MHCII molecule and stimulating a response from SAg-specific T cells without further antigen processing. Variants of peptide 15N3K (SEQ ID NO: 3) were tested for their ability to bind to an MHCII molecule and be presented to a T cell hybridoma without further antigen processing in an *in vitro* antigen processing independent presentation system (APIPS) assay. In other words, peptides consisting of parts of peptide 15N3K and peptides with various degrees of sequence identity with peptide 15N3K were tested for their ability to behave as apitopes.

The peptides tested are presented in the table below:

| **Name** | **SEQ ID NO** | **Sequence** | **Apitope** |
|---|---|---|---|
| HIP-115 | 5 | MAASGKTSKSEPNHVIFKKISRDKSVTIYLGNRDYIDHVSQV | Yes |
| 15N3K | 3 | KKKVIFKKISRDKSVTIYLGKKK | Yes |
| HIP-15B1 | 6 | VIFKKISRDKSVTI | Yes |
| HIP-15B1-KKK | 7 | KKKVIFKKISRDKSVTIKKK | Yes |
| HIP-15E1 | 8 | KKISRDKSVTIYLG | Yes |
| HIP-15E1-KKK | 9 | KKKKKISRDKSVTIYLGKKK | Yes |
| HIP-15CD | 10 | IFKKISRDKSVTIYLG | Yes |
| HIP-15CD-KKK | 11 | KKKIFKKISRDKSVTIYLGKKK | Yes |
| HIP-15BC | 12 | VIFKKISRDKSVTIYL | Yes |
| HIP-15BC-KKK | 13 | KKKVIFKKISRDKSVTIYLKKK | Yes |
| HIP-15B3 | 14 | VIFKKISRDKSV | No |
| HIP-15B3-KKK | 15 | KKKVIFKKISRDKSVKKK | No |
| HIP-15G3 | 16 | ISRDKSVTIYLG | No |
| HIP-15G3-KKK | 17 | KKKISRDKSVTIYLGKKK | Yes |

The response of T cells, as measured by IL-2 secretion, to each of these peptides in the APIPS assay is shown in Figure 10. All but three of the peptides are apitopes.

### Materials & Methods

### Mice

Throughout the peptide identification and apitope development HLA-DR transgenic mice was used to ensure the peptide-MHC class II binding motif is exactly as required for tolerization treatment in uveitis patients.

DR3tg mice were bred under specific pathogen-free conditions externally at Charles River, UK, or at Innoser, Belgium. The DR3tg strain was originally created by Strauss et al (Strauss et al, 1994, Immunogenetics 3, 104-108). In brief, the genomic constructs used were a 6 kb Ndel fragment of a HLA-DRA genomic clone in pUC 13 and a 24 kb C*la*I*xSal*I fragment of cos 4.1, a cosmid (pTCF) containing the 8 gene of DRB1*0301. A solution containing 1-2 µg/mL of each construct was used for co-injection into fertilised eggs from (C57BL/6 x DBA/2) F1 donors mated with C57BL/6 males. The offspring has later been bred into the IA-beta knockout C57BL/6 genetic background (AB0 mice) lacking mouse MHC class II molecule expression. These DR3tg mice express the HLA-DRB1*0301 molecule but not the mouse MHC-II molecule. The mice were maintained by backcrossing to C57BL/6 and to B10.Q. Transgenic mice were identified by Southern blot analysis of tail DNA digested with *EcoR*I and probed with a 1.35 kb *BamH*I fragment of the DRA cDNA and a 1.25 kb *BamH*I fragment of the DRB1*0301 cDNA

DR2tg mice were bred under specific pathogen-free conditions externally at Charles River, UK, or at Innoser, Belgium. HLA-DR2 transgenic (DR2tg) mice were originally obtained from Lars Fugger (Madsen et al., 1999). In brief, DRα and DRβ chain cDNAs (DRA*0101 and DRB1*1501) were expressed by using the pDOI-5 expression vector which contains a mouse MHCII promotor. The constructs were injected into fertilised eggs from (DBA/2xC57BL/6)F1 matings. The mice were backcrossed into the IA-beta knockout C57BL/6 genetic background (AB0 mice) lacking mouse MHC class II molecule expression. The DR2tg mice express the HLA-DRB1*1501 molecule but not the mouse MHC molecule.

Animal studies were approved by the 'Ethical Committee for Animal experiments' (ECD) at Hasselt University and performed with the highest standards of care in a pathogen-free facility

### Antigens

All single peptides were synthesized by Genscript (Piscataway, USA) and stored at a stock solution of 20 mg/ml in DMSO (Sigma-Aldrich) or 4 mg/ml in PBS (Lonza) at - 80°C. The peptides were synthesized with an N-terminal free amine and a C-terminal amide. Human S-Ag (S-arrestin) was produced in HEK293F cells (QBiologicals, Eurofins Amatsigroup, Ghent, Belgium).

### In vivo MHC class II loading assay

DR3tg or DR2tg mice were injected with 100 µg of peptide in 100 µl PBS subcutaneously (s.c.) in the flank. Control animals received a s.c. injection of 100 µl PBS. After 2 hours, spleens were harvested and single-cell suspensions were prepared. CD11c+ cells were positively selected using CD11c microbeads according to the manufacturer's instructions (Miltenyi Biotec, Bergisch Gladbach, Germany). Average purities of >92% were reached. 1×10⁵ CD11c+ cells were co-cultured with 1×10⁵ CD4+ cells in round bottom 96-well plates in X-vivo 15 medium (supplemented with 2mM L-glutamine, 50 U/mL penicillin and 50 U/mL streptomycin; Lonza and 50 mM β-mercaptoethanol; Gibco). These CD4+ cells were isolated from DR3tg or DR2tg mice that were immunised subcutaneously in the base of the tail with 50 µg peptide emulsified in CFA (peptide/CFA). Ten days after immunisation, draining lymph nodes (LN) and spleens were harvested. LN cells and splenocytes were isolated and CD4+ T cells were isolated by negative selection using Magnisort Mouse CD4 Isolation kit (ThermoFisher Scientific) according to the manufacturer's instructions. After 72 hours, supernatant of these co-cultures was collected and CD4+ T cell activation was analysed by IFNγ ELISA (R&D Systems, Abingdon, UK). In a parallel experiment, CD4+ T cell responses towards peptide added in vitro were assessed to make sure the T cells recognize the peptides presented by the CD11c+ cells.

### Ex vivo tolerization experiment

DR3tg or DR2tg mice were injected subcutaneously in the flank region with 0.015 nmol, 0.15 nmol and 1.5 nmol of peptide on days -15, -13 and -11 respectively(dose escalation schedule), followed by 3 injections of 15 nmol peptide on days -8, -6 and - 4.Indicated doses are for single peptide treatments, for treatment with the cocktail containing the 3 peptides included in ATX975, these doses were given per peptide (reaching a total highest dose of 45 nmol peptide). Control mice received the same dose of a non-relevant peptide able to bind HLA-DR2 or HLA-DR3, depending on the mouse strain used. On day 0, the mice were immunised subcutaneously in the base of the tail with 150 µg antigen (50 µg of each 30-mer peptide containing the respective epitope) emulsified in CFA (peptide/CFA). Ten days after immunisation, draining lymph nodes (LN) and spleens were harvested. LN cells and splenocytes were isolated and cultured in X-vivo 15 medium (supplemented with 2mM L-glutamine, 50 U/mL penicillin and 50 U/mL streptomycin; Lonza and 50 mM β-mercaptoethanol; Gibco) in 96-well round bottom plates. To investigate antigen-induced cell activation, 0.5×10⁶ cells/well were cultured (200 µl/well) for 72 hours with different antigen concentrations (0-25 µg/ml) or with 12.5 µg/ml purified protein derivative (PPD; priming control; AJ Vaccines, Copenhagen, Denmark). After 72 hours, supernatant was harvested and stored at -80°C until further analysis. IFN-γ concentrations in supernatants were assessed by cytokine ELISA (R&D Systems, Abingdon, UK) and cell activation was measured.

### Peptide Sequences

| **Peptide** | **Sequence** | **SEQ ID NO:** |
|---|---|---|
| 9K1K | KKKAFVEQVANWLKKK | SEQ ID NO: 1 |
| 17JK | KKKLTFRRDLYFSRVQVYKKK | SEQ ID NO: 2 |
| 15N3K | KKKVIFKKISRDKSVTIYLGKKK | SEQ ID NO: 3 |

### Peptide-MHC class II Binding Assay

Binding of peptides 9K1, 17JK and 15N3K to recombinant HLA-DRA1*0101, DRB1*0101 (DR1), DRB1*1501 (DR2), DRB1*0301 (DR3), DRB1*0401 (DR4), DRB1*1101 (DR11), DRB1*0405 (DR4*05) and DRB1*0901 (DR9) was performed by Prolmmune (Oxford, UK) using their cell-free MHC Class II REVEAL binding assay.

### Antigen Processing Independent Presentation System (APIPS) Assay

Antigen-specific hybridoma clones were tested for their reactivity to the peptides, presented by fixed or not fixed (fresh) cells (APCs). 5×10⁴ cells were cultured with 10 µg/ml and 25 µg/ml peptide and 5×10⁴ fixed or fresh APCs. To fix APCs, cells were incubated with 0.5% paraformaldehyde (Merck, Darmstadt, Germany) (pH7) for 5 min at room temperature (RT). The fixation reaction was stopped by adding 0.4M glycine (Sigma-Aldrich) and washing the cells in RPMI-10% FCS. After 48h, antigen-induced IL-2 production was measured by ELISA (R&D Systems, Abingdon, UK).

## Claims

1. A composition which comprises S-arrestin (S-Ag) peptides, wherein the S-Ag peptides consist of the following S-Ag peptides:
a peptide consisting of the amino acid sequence KKKAFVEQVANWLKKK (SEQ ID NO: 1); and
a peptide consisting of the amino acid sequence KKKLTFRRDLYFSRVQVYKKK (SEQ ID NO: 2); and
a peptide consisting of the amino acid sequence KKKVIFKKISRDKSVTIYLGKKK (SEQ ID NO: 3).

2. The composition according to claim 1 wherein the peptides are capable of binding to an MHC molecule *in vitro* and being presented to a T cell without antigen processing.

3. The composition according to claim 1 or claim 2, for use in therapy.

4. The composition according to claim 1 or claim 2, for use in treating and/or preventing uveitis in a subject.

5. The composition for use according to claim 3 or claim 4, wherein the subject expresses HLA-DR3.

6. The composition for use according to claim 3 or claim 4, wherein the subject expresses HLA-DR2.

7. The composition for use according to any of claims 3 to 6, in which the composition is administered following a dose-escalation protocol.

8. The composition for use according to claim 7, wherein the dose-escalation protocol comprises the following doses:
Day 1: a first dose of about 15 to about 40µg;
Day 14±7 days: a second dose of about 35-65µg;
Day 28±7 days: a third dose of about 80-120µg;
Day 42±7 days: a fourth dose of about 300-500µg;
Day 56±7 days: a fifth dose of about 600-1500µg;
Day 70±7 days: a sixth dose of about 600-1500µg;
Day 84±7 days: a seventh dose of about 600-1500µg;
Day 98±7 days: an eighth dose of about 600-1500µg;
Day 112±7 days: a ninth dose of about 600-1500µg; and
Day 126±7 days: a tenth dose of about 600-1500µg.

9. The composition for use according to claim 8, wherein
the first dose is about 25µg; and/or
the second dose is about 50µg; and/or
the third dose is about 100µg; and/or
the fourth dose is about 400µg.

10. The composition for use according to claim 8 or claim 9, wherein
an eleventh dose of about 600-1500µg is administered on day 140±7 days; and/or
a twelfth dose of about 600-1500µg is administered on day 154±7 days; and/or
a thirteenth dose of about 600-1500µg is administered on day 168±7 days.

11. The composition for use according to any of claims 8 to 10 wherein the fifth, sixth, seventh, eighth, ninth and tenth, and optionally eleventh, twelfth and thirteenth, doses are each about 800µg.

12. The composition for use according to any of claims 3 to 11 wherein administration of the composition is intradermal.

13. The composition for use according to any of claims 3 to 12 wherein the composition is administered to a human.

14. A kit which comprises the S-Ag peptides as defined in claim 1 or claim 2.

15. The kit of claim 14 for simultaneous, separate or sequential administration in the prevention or treatment of uveitis.

## Patentansprüche

1. Zusammensetzung, die S-Arrestin(S-Ag)-Peptide umfasst, wobei die S-Ag-Peptide aus den folgenden S-Ag-Peptiden bestehen:
einem Peptid, das aus der Aminosäuresequenz KKKAFVEQVANVVLKKK (SEQ ID NO: 1) besteht; und
einem Peptid, das aus der Aminosäuresequenz KKKLTFRRDLYFSRVQVYKKK (SEQ ID NO: 2) besteht; und
einem Peptid, das aus der Aminosäuresequenz KKKVIFKKISRDKSVTIYLGKKK (SEQ ID NO: 3) besteht.

2. Zusammensetzung nach Anspruch 1, wobei die Peptide *in vitro* an ein MLC-Molekül binden und ohne Antigenprozessierung einer T-Zelle präsentiert werden können.

3. Zusammensetzung nach Anspruch 1 oder Anspruch 2 zur therapeutischen Verwendung.

4. Zusammensetzung nach Anspruch 1 oder Anspruch 2, zur Verwendung bei der Behandlung und/oder Vorbeugung von Uveitis bei einem Individuum.

5. Zusammensetzung zur Verwendung nach Anspruch 3 oder Anspruch 4, wobei das Individuum HLA-DR3 exprimiert.

6. Zusammensetzung zur Verwendung nach Anspruch 3 oder Anspruch 4, wobei das Individuum HLA-DR2 exprimiert.

7. Zusammensetzung zur Verwendung nach einem der Ansprüche 3 bis 6, wobei die Zusammensetzung nach einem Dosiseskalationsprotokoll verabreicht wird.

8. Zusammensetzung zur Verwendung nach Anspruch 7, wobei das Dosiseskalationsprotokoll die folgenden Dosen umfasst:
Tag 1: eine erste Dosis von etwa 15 bis etwa 40 µg;
Tag 14±7 Tage: eine zweite Dosis von etwa 35-65 µg;
Tag 28±7 Tage: eine dritte Dosis von etwa 80-120 µg;
Tag 42±7 Tage: eine vierte Dosis von etwa 300-500 µg;
Tag 56±7 Tage: eine fünfte Dosis von etwa 600-1500 µg;
Tag 70±7 Tage: eine sechste Dosis von etwa 600-1500 µg;
Tag 84±7 Tage: eine siebte Dosis von etwa 600-1500 µg;
Tag 98±7 Tage: eine achte Dosis von etwa 600-1500 µg;
Tag 112±7 Tage: eine neunte Dosis von etwa 600-1500 µg; und
Tag 126±7 Tage: eine zehnte Dosis von etwa 600-1500 µg.

9. Zusammensetzung zur Verwendung nach Anspruch 8, wobei
die erste Dosis etwa 25 ug beträgt; und/oder
die zweite Dosis etwa 50 ug beträgt; und/oder
die dritte Dosis etwa 100 µg beträgt und/oder
die vierte Dosis etwa 400 ug beträgt.

10. Zusammensetzung zur Verwendung nach Anspruch 8 oder Anspruch 9, wobei
eine elfte Dosis von etwa 600-1500 µg an Tag 140±7 Tage verabreicht wird; und/oder
eine zwölfte Dosis von etwa 600-1500 µg wird an Tag 154±7 Tage verabreicht wird; und/oder
eine dreizehnte Dosis von etwa 600-1500 µg an Tag 168±7 Tage verabreicht wird.

11. Zusammensetzung zur Verwendung nach einem der Ansprüche 8 bis 10, wobei die fünfte, sechste, siebte, achte, neunte und zehnte und gegebenenfalls die elfte, zwölfte und dreizehnte Dosis jeweils etwa 800 µg betragen.

12. Zusammensetzung zur Verwendung nach einem der Ansprüche 3 bis 11, wobei die Verabreichung der Zusammensetzung intradermal erfolgt.

13. Zusammensetzung zur Verwendung nach einem der Ansprüche 3 bis 12, wobei die Zusammensetzung einem Menschen verabreicht wird.

14. Kit, das die S-Ag-Peptide gemäß Anspruch 1 oder Anspruch 2 umfasst.

15. Kit gemäß Anspruch 14 zur gleichzeitigen, separaten oder sequenziellen Verabreichung bei der Vorbeugung oder Behandlung von Uveitis.

## Revendications

1. Composition comprenant des peptides de S-arrestine (S-Ag), les peptides S-Ag étant constitués des peptides S-Ag suivants :
un peptide constitué de la séquence d'acides aminés KKKAFVEQVANVVLKKK (SEQ ID NO : 1) ; et
un peptide constitué de la séquence d'acides aminés KKKLTFRRDLYFSRVQVYKKK (SEQ ID NO : 2) ; et
un peptide constitué de la séquence d'acides aminés KKKVIFKKISRDKSVTIYLGKKK (SEQ ID NO : 3).

2. Composition selon la revendication 1, dans laquelle les peptides sont capables de se lier à une molécule du CMH *in vitro* et d'être présentés à un lymphocyte T sans traitement antigénique.

3. Composition selon la revendication 1 ou la revendication 2, destinée à être utilisée en thérapie.

4. Composition selon la revendication 1 ou la revendication 2, destinée à être utilisée dans le traitement et/ou la prévention de l'uvéite chez un sujet.

5. Composition destinée à être utilisée selon la revendication 3 ou la revendication 4, le sujet exprimant HLA-DR3.

6. Composition destinée à être utilisée selon la revendication 3 ou la revendication 4, le sujet exprimant HLA-DR2.

7. Composition destinée à être utilisée selon l'une quelconque des revendications 3 à 6, la composition étant administrée selon un protocole d'escalade de dose.

8. Composition destinée à être utilisée selon la revendication 7, le protocole d'escalade de dose comprenant les doses suivantes :
Jour 1 : première dose d'environ 15 à 40 µg ;
Jour 14 ± 7 jours : deuxième dose d'environ 35 à 65 µg ;
Jour 28 ± 7 jours : troisième dose d'environ 80 à 120 µg ;
Jour 42 ± 7 jours : quatrième dose d'environ 300 à 500 µg ;
Jour 56 ± 7 jours : cinquième dose d'environ 600 à 1 500 µg ;
Jour 70 ± 7 jours : sixième dose d'environ 600 à 1 500 µg ;
Jour 84 ± 7 jours : septième dose d'environ 600 à 1 500 µg ;
Jour 98 ± 7 jours : huitième dose d'environ 600 à 1 500 µg ;
Jour 112 ± 7 jours : neuvième dose d'environ 600 à 1 500 µg ; et
Jour 126 ± 7 jours : dixième dose d'environ 600 à 1 500 µg.

9. Composition destinée à être utilisée selon la revendication 8, dans laquelle
la première dose est d'environ 25 µg ; et/ou
la deuxième dose est d'environ 50 µg ; et/ou
la troisième dose est d'environ 100 µg ; et/ou
la quatrième dose est d'environ 400 µg.

10. Composition destinée à être utilisée selon la revendication 8 ou la revendication 9, dans laquelle une onzième dose d'environ 600 à 1 500 µg est administrée au jour 140 ± 7 jours ; et/ou
une douzième dose d'environ 600 à 1 500 µg est administrée au jour 154 ± 7 jours ; et/ou
une treizième dose d'environ 600 à 1 500 µg est administrée au jour 168 ± 7 jours.

11. Composition destinée à être utilisée selon l'une quelconque des revendications 8 à 10, les cinquième, sixième, septième, huitième, neuvième et dixième, et éventuellement onzième, douzième et treizième, doses étant chacune d'environ 800 µg.

12. Composition destinée à être utilisée selon l'une quelconque des revendications 3 à 11, l'administration de la composition étant intradermique.

13. Composition destinée à être utilisée selon l'une quelconque des revendications 3 à 12, la composition étant administrée à un humain.

14. Kit comprenant les peptides S-Ag tels que définis dans la revendication 1 ou la revendication 2.

15. Kit selon la revendication 14 pour administration simultanée, séparée ou séquentielle dans la prévention ou le traitement de l'uvéite.
